# EUROPEAN PATENT SPECIFICATION

(11) **EP 4 291 867 B1**
(45) Date of publication and mention of the grant of the patent: **18.12.2024**
(21) Application number: 22704574.7
(22) Date of filing: 11.02.2022
(51) Int. Cl.: G01N 13/00, G01N 33/543, G01N 33/58, G01N 1/40

(54) **IMPROVEMENTS IN OR RELATING TO A METHOD OF ANALYSING A COMPONENT IN A SAMPLE**
VERBESSERUNGEN AN ODER IM ZUSAMMENHANG MIT EINEM VERFAHREN ZUR ANALYSE EINER KOMPONENTE IN EINER PROBE
AMÉLIORATIONS APPORTÉES À UN PROCÉDÉ D'ANALYSE D'UN COMPOSANT DANS UN ÉCHANTILLON

(30) Priority: 12.02.2021 GB 202101979
(43) Date of publication of application: 20.12.2023
(73) Proprietor: Cambridge Enterprise Limited, Cambridge, Cambridgeshire CB2 1TN (GB)
(72) Inventor: KNOWLES, Tuomas Pertti Jonathan, Cambridge Cambridgeshire CB2 1TN (GB); JACQUAT, Raphael Philippe Bernard, Cambridge Cambridgeshire CB2 1TN (GB); MUELLER, Thomas, 6340 Baar (CH); PETER, Quentin, 6340 Baar (CH)
(74) Representative: Stratagem IPM Limited
(86) International application number: PCT/GB2022/050375
(87) International publication number: WO 2022/172023

(56) References cited:
- ANONYMOUS: "What is Microfluidic Diffusional Sizing (MDS)? - Fluidic Analytics", FLUIDIC ANALYTICS CORPORATE WEBSITE (HTTPS://FLUIDIC.COM/), 10 September 2019 (2019-09-10), Cambridge (United Kingdom), XP055856060, Retrieved from the Internet <URL:https://www.fluidic.com/resources/what-is-microfluidic-diffusional-sizing/> [retrieved on 20211028]
- AZOUZ MEHDI ET AL: "Microfluidic diffusional sizing probes lipid nanodiscs formation", BBA - BIOMEMBRANES ACTA, ELSEVIER, AMSTERDAM, NL, vol. 1862, no. 6, 20 February 2020 (2020-02-20), XP086106578, ISSN: 0005-2736, [retrieved on 20200220], DOI: 10.1016/J.BBAMEM.2020.183215
- LINSE SARA ET AL: "Kinetic fingerprints differentiate the mechanisms of action of anti-A[beta] antibodies", NAT. STRUCT. MOL. BIOL, vol. 27, no. 12, 28 September 2020 (2020-09-28), pages 1125 - 1133, XP037311090, ISSN: 1545-9993, DOI: 10.1038/S41594-020-0505-6
- APRILE FRANCESCO A ET AL: "Rational design of a conformation-specific antibody for the quantification of A[beta] oligomers", PROCEEDINGS OF THE NATIONAL ACADEMY OF SCIENCES - PNAS, 16 June 2020 (2020-06-16), United States, pages 13509 - 13518, XP055856065, Retrieved from the Internet <URL:https://www.pnas.org/content/pnas/117/24/13509.full.pdf> [retrieved on 20211028], DOI: 10.1073/pnas.1919464117
- SCHNEIDER MATTHIAS M. ET AL: "Microfluidic Affinity Profiling reveals a Broad Range of Target Affinities for Anti-SARS-CoV-2 Antibodies in Plasma of Covid Survivors", MEDRXIV, 20 September 2020 (2020-09-20), pages 1 - 14, XP055856080, Retrieved from the Internet <URL:https://www.medrxiv.org/content/10.1101/2020.09.20.20196907v1.full.pdf> [retrieved on 20211028], DOI: 10.1101/2020.09.20.20196907
- SCHNEIDER MATTHIAS M. ET AL: "Microfluidic Antibody Affinity Profiling for In-Solution Characterisation of Alloantibody - HLA Interactions in Human Serum", BIORXIV, 3 November 2020 (2020-11-03), pages 1 - 14, XP055856138, Retrieved from the Internet <URL:https://www.biorxiv.org/content/10.1101/2020.09.14.296442v2.full.pdf> [retrieved on 20211028], DOI: 10.1101/2020.09.14.296442
- YATES EMMA V., MÜLLER THOMAS, RAJAH LUKE, DE GENST ERWIN J., AROSIO PAOLO, LINSE SARA, VENDRUSCOLO MICHELE, DOBSON CHRISTOPHER M.: "Latent analysis of unmodified biomolecules and their complexes in solution with attomole detection sensitivity", NATURE CHEMISTRY | VOL. 7 | OCTOBER 2015, vol. 7, 14 September 2015 (2015-09-14), London (UK), pages 802 - 809, XP055789144, Retrieved from the Internet <URL:http://www.nature.com/articles/nchem.2344> [retrieved on 20211029], DOI: 10.1038/nchem.2344
- FLUIDIC: "How does the Fluidity One-W work?", YOUTUBE, 12 September 2019 (2019-09-12), XP054982406, Retrieved from the Internet <URL:https://www.youtube.com/watch?v=HyWR_QIy9no> [retrieved on 20211029]

## Description

The present invention relates to a method for analysing a biophysical property of a component in a sample and, in particular, a method of determining a biophysical property of two or more components in a polydisperse sample.

Protein-protein interactions form the basis of many biologically and physiologically relevant processes including: protein self-assembly; protein-aggregation; antibody-antigen recognition; muscle contraction and cellular communication. Nevertheless, studying protein-protein interactions, especially under physiological conditions in complex media, remains challenging. Current techniques, such as an enzyme-linked immunosorbent assay (ELISA) bead-based multiplex assay and surface plasmon resonance (SPR) spectroscopy, rely on immobilisation of one binding partner. These techniques include potential unspecific interactions with the surface, which can cause false positive results and the Hook/Prozone effect, which causes false-negative results, thereby allowing semi-quantitative analysis only.

Microfluidic devices have been previously deployed to separate components in a fluid flow within a sample in a channel. The flow can then be split into further downstream flows where the sample is further labelled. Electrokinetic separation techniques, such as capillary zone electrophoresis (CE) capillary gel electrophoresis (CGE), capillary isoelectric focusing (ClEF), capillary isotachophoresis and micellar electrokinetic chromatography (MEKC) are powerful analytical tools commonly used to separate a plurality of components in a sample in solution. Separation of components using CE is based on the electrophoretic mobility of the component and thus CE allows for determination of purity of components in a sample.

The separated samples can then be detected optically with absorption, scattering or fluorescence measurements. Alternatively, the separated samples can be determined electrically or electrochemically as well as off chip after collection or injection into a down-stream detection module.

The ability to label the component means that the components in the flows can be detected using high resolution detection techniques, such as fluorescence, and analysed to provide information on the biophysical properties of the component, such as the size of the components within the sample. The ability to label a component after fractionation and/or separation allows for the fractionation and/or separation to happen based on the unlabelled state.

However, previous techniques can only provide an average size of the component i.e. the radius of the component in the sample. This is particularly relevant for components that are part of a multicomponent mixture, in which the proportion of each component in the lateral distribution is representative of the relative proportions of those components in the native state. This may not provide the most accurate information on each individual separated component within the sample. Therefore, there is a requirement to provide a method for analysing the separated components independently in order to obtain the radius for each component in the sample.

An example of such a method is disclosed in AZOUZ MEHDI ET AL: "Microfluidic diffusional sizing probes lipid nanodiscs formation", BBA - BIOMEMBRANES ACTA, ELSEVIER, AMSTERDAM, NL, vol. 1862, no. 6, 20 February 2020 (2020-02-20), ISSN: 0005-2736, DOI: 10.1016/J.BBAMEM.2020.183215.

It is against the background that the present invention has arisen. According to 2. the claimed em invention, there is provided a method of determining the diffusion coefficient of one or more components in a polydisperse sample, the method comprising the following steps in the following order: introducing an auxiliary fluid flow into a fractionation channel; introducing the polydisperse sample comprising one or more components into the fractionation channel; allowing the sample fluid and the auxiliary fluid to create a combined flow; fractionating the combined flow into two or more fractions by diffusive sizing; adding into each fraction solutes or solvents selected from: affinity probes or immunoprobes for labelling; ions or salts to perform pre-concentration; additives to reduce risk of sample adhesion to the wall of the channel of the microfluidic device; additives to modify the amount of the electro-osmotic flow within an electrophoresis separation channel; additives to enhance the quantum yield or lifetime of a fluorophore; separating two or more components from each fraction by creating a distribution of the components within a separation channel; processing the components in one or more of the fractions to separate and detect components added before or during the method, wherein said added components include: a blank or auxiliary fluid; any additive such as immunoprobes or affinity probes; ions or salts to perform pre-concentration; additives to modify the amount of the electro-osmotic flow within an electrophoresis separation channel; additives to enhance the quantum yield or lifetime of a fluorophore; detecting a characteristic of each of the two or more components in each fraction; and comparing the characteristic of each component in each fraction in order to determine the diffusion coefficient of each of the one or more components in the polydisperse sample.

In some embodiments, the separation step includes separation of at least one fraction and at least one reference fraction, wherein the reference fraction is either another fraction from diffusive sizing and/or the pre-fractionation sample. It may be possible to get the same information from this comparison as comparing diffused and not-diffused fractions by comparing diffused and non-fractionated sample, because the unfractionated sample includes the diffused and undiffused samples, divided by a dilution factor. The dilution factor is often two.

The method of the present invention as disclosed herein can be used for separation and analysis of a component in a sample by first dividing a sample into fractions and then separating the constituent components of the fractions using a high-resolution separation technique.

A sample fluid flow can be in a steady state flow during fractionation. The sample does not need to be completely separated after fractionation, but a difference in relative or absolute concentration of the components must exist.

The separation of one or more components in a polydisperse sample can be particularly useful for obtaining biophysical properties of each separated component such as size and electrophoretic mobility, instead of having weighted average information. As disclosed herein, and unless otherwise specified, the term polydisperse refers to a sample of one or more components.

The sample flow can be decoupled, enabling different conditions for fractionation and separation steps. Furthermore, the method of present invention allows for the fractionation of native species by processing each fraction, for example adding the label or immunolabel, after the fractionating step. Thus, the ability of analysing the separated components independently can provide accurate analysis of biophysical property such as size or hydrodynamic radius for each separated component.

In some instances, it can be particularly useful to determine the ratio between species that are bound and not bound to another molecule in order to identify the component of interest in a polydisperse sample. For example, the polydisperse sample can be provided with an affinity probe or an immunoprobe which can have a specific affinity to a single component or multiple components of interest. The probe may be labelled. A known quantity of an affinity probe or an immunoprobe would be added to the polydisperse sample. The method of the present invention as disclosed herein can then be used to determine the ratio between the unbound and bound affinity probe and/or the immunoprobe within the sample. This, in turn, can be used to determine the quantity and/or affinity of the one or more components of interest in the polydisperse sample to the affinity probe.

In some embodiments, multiple affinity probes can be used to detect and/or quantify multiple components of interest as well as determine their affinity to the multiple affinity probes.

In some embodiments, the method according to the present invention is based on entropy-based fractionation method such as diffusional sizing.

The fractionation step can be done by diffusional sizing. The fractions are usually incompletely separated and most components will be present in every fraction at different concentrations. In some embodiments, the fractionation step of the combined flow into two or more fractions can be an entropy-driven separation. Diffusional sizing is an example of entropy-driven separation technique.

Entropy-based fractionation techniques distribute the sample into two or more fractions. These fractions may typically contain all or most of the components from the original sample but with different relative concentrations. It is therefore necessary to analyse the differences in relative concentrations thereafter. After the fractionation step, each fraction can then collected separately for processing, further separation and analysis. Alternatively or additionally, one or more fractions can also be compared to the original sample upon processing, further separation and analysis of the fraction and the original sample. The advantage of this is that the concentration of the original sample is typically higher than that of a fraction.

The detection techniques deployed may be selected, as appropriate from various different approaches to the detection of electromagnetic radiation of various types. The detection techniques may include, but are not limited to, the following:
∘ Optical detection, across any suitable waveband, not limited to the visible, but including X-ray, UV and IR as applicable
   ▪ Fluorescence such as detecting the auto-fluorescence or intrinsic fluorescence of the component or detecting the fluorescence of a dye attached onto the component;
   ▪ Chemiluminescence;
   ▪ Absorbance;
   ▪ Scattering;
∘ Electrical detection
   ▪ Conductivity detection;
   ▪ Charge detection;
   ▪ Electrochemical detection;
∘ Magnetic detection
   ▪ Magnetic moment or force detection; or
∘ Mass measurement
∘ Detection of radioactivity from radioactive labels

The detector can be used to measure the number of components, concentration of the component, flux of component (concentration X velocity) in each fraction.

The separation of one or more components in each fraction can be used to enable a comparison between each fraction and/or between a fraction and the original sample. This comparison enables the extraction of inaccessible information, such as the size of each component, from the fractionation step. Without separation, only a single, weighted average size of the mixture of components is accessible. These results can be used to determine a property of the sample under the condition of the first fractionation step of the sample fluid flow.

The separation channel can be downstream from the fractionation channel and may be fluidically connected. In some embodiments, the fractionation channel and the separation channel is not required to be fluidically connected. The fractionation channel may contain a collection well at its downstream ends to collect the fractions.

In some embodiments the fractionation and separation channels are on microfluidic chips. Additionally or alternatively, they may be on the same chip. Alternatively, they may be on a separate chip. Alternatively, the separation may be done on a separate micro-capillary.

Alternatively, a different fluid such as a blank fluid can be used for priming the fluids in the device prior to loading the sample fluid into the device. Loading a blank fluid flow into the device can help to reduce or even to avoid bubbles forming within the channels of the device. The blank fluid flow can be a buffer solution.

Sources of background signal may include, but is not limited to; fluorescence, absorption, reflections and scattering from the sample and auxiliary fluids, chip materials and wider opto-mechanical system. Furthermore, different system fluids may have different background signal level. For example, a protein in water has low background signal due to the lack of fluorescent components causing a spurious detection signal, but water may not be appropriate as a sample or system fluid since it is not buffered. Instead, it may be preferable to match the auxiliary fluid and the sample fluid so that, for example, differences in viscosity do not impact the readings. As a result, it may be preferable to use serum as the auxiliary fluid, despite the fact that serum has a high level of background. Alternatively it might be advantageous to use a buffered solution mimicking the properties of serum as the auxiliary fluid. The properties to match may be a selection of one or more of: viscosity, salt concentration, total protein concentration. Furthermore, it may be advantageous to match the refractive index between sample fluid and microfluidic chip material and/or the immersion material of the objective lens of an optical detection system.

As disclosed herein, the method comprises the step of comparing the characteristic of each component in each fraction in order to determine the biophysical property of each of the one or more components in the polydisperse sample. In some embodiments, the method may further comprise the step of comparing the quantities, amounts of or the number of molecules of each component in each fraction in order to determine the biophysical property of each of the one or more components in the sample. Alternatively or additionally, the method may comprise the step of comparing the quantities, amounts of or the number of molecules of each component in one or more fractions with the quantities, amounts of or the number of molecules of each component in the original sample in order to determine the biophysical property of each of the one or more components in the sample.

The biophysical property of the component can be the size and particularly the hydrodynamic radius of the component. In some embodiments, the biophysical property of the component can be diffusion coefficient of the component. In some embodiments, the biophysical property of the component can be charge of the component.

In some embodiments, the method may further comprise the step of processing the components in one or more fractions. In some embodiments, the method may further comprise the step of processing the components in an aliquot of the original sample. In some embodiments, the components in one or more fractions can be further processed after fractioning the combined fluid flow. For example, according to the claimed invention it is foreseen to add into each fraction solutes or solvents selected from:
- Affinity probes or Immunoprobes for labelling
- Ions or salts to perform pre-concentration
- Additives to reduce risk of sample adhesion to the wall of the channel of the microfluidic device
- Additives to modify the amount of the electro-osmotic flow within an electrophoresis separation channel
- Additives to enhance the quantum yield or life time of a fluorophore

The added solutes and/or solvents can affect the molecules, particles or concentrations of the component. Therefore, performing this step first could make it impossible to retrieve the desired information. However, performing the processing step after the fractionation step is advantageous as it preserves the related biophysical properties and information of the component, even if the components are modified.

According to the claimed invention, the method may further comprise the step of processing the components in one or more of the fractions to separate and detect components added before or during the method. This separation can improve the signal quality. In particular, removing the contributions of these components from the final profile will isolate the contribution of the species of interest. Components that can be separated in this manner include according to the claimed invention a blank or auxiliary fluid; any additives such as immunoprobes or affinity probes; ions or salts to perform pre-concentration; additives to reduce risk of sample adhesion to the wall of the channel of the microfluidic device; additives to modify the amount of the electro-osmotic flow within an electrophoresis separation channel; and/or additives to enhance the quantum yield or life time of a fluorophore.

Alternatively, it may be possible to use a concentration assay to increase the absolute and/or relative concentration of the species of interest and their binding partners, such as a pull-down or immunoprecipitation assay using magnetic or polymer beads or affinity columns. The component may be eluted on its own or together with the capture probe or bait. Typically, upon elution the component may be altered due to denaturation, digestion, or being bound to a capture probe. Additionally or alternatively, a pull-down or immunoprecipitation assay allows for washing out unwanted components from the sample mixture.

The high-resolution separation of each fraction can be performed in series or in parallel at the same time. The result of this separation step can be transient and a measurement can be taken.

In some embodiments, fractions can be collected, stored and measured at a later date which can provide more flexibility in sample analysis. One of the advantages of performing high resolution separation is the fact that a high number of very small separation fractions can be processed quickly to keep the separated components apart. The measurement can be done by a different technique.

In some embodiments, the method may further comprise the step of processing the component in the polydisperse sample prior to fractionating the combined flow. In some embodiments, the processing step prior to the fractionation step may include, but is not limited to, the step of concentrating the one or more components of interest in the polydisperse sample prior to fractionation using a pull down assay or immunoprecipitation.

Fractions can be collected in parallel which can be advantageous as there are no time constraints. Additionally or alternatively, fractions can also be collected in series.

In some embodiments, the processing step may comprise the step of labelling the components in each fraction.

Labelling the component enables the detection of the component in each fraction. Examples of labelling may include, but is not limited to, the following: Immunolabelling, such as attaching a labelled immune-probe, including antibodies, antibody fragments, single-chain antibodies, aptamers; fluorescent labels, FRET pair labelling and/or a combination thereof.

These labels can be specific to a sub-set of the components for example, labelled immuno-probes for "fishing" out targets from a complex mixture such as a blood plasma sample.

In some embodiments, the labelling of the component comprises an affinity probe. In some embodiments, the affinity probe is labelled.

An affinity probe is a probe with a specific affinity to one or more components. In some embodiments, the affinity probe can be one or more of the following; an antibody, antibody fragment(s) thereof or a nanobody. In some embodiments, the affinity probe can be an aptamer or a darpin.

In some embodiments, the label of the components in each fraction can be a fluorescent label.

In some embodiments, the label of the components in each fraction can be a non-latent fluorescence label. Non-latent labels can be specific to a sub-set of the components for example, labelled immuno-probes for "fishing" out targets from a complex mixture such as blood plasma.

In some embodiments, the fluorescent labelling of the two or more components in the polydisperse sample can occur prior to the fractionation step. Labelling before the fractionation step results in a simpler workflow with the labelling of a single sample, rather than a number of different flows requiring the label. Conversely, labelling after the fractionation, before the separation, allows the fractionation step to take place on the basis of the unlabelled sample.

The separation and analysis are performed in solution, and even pre-fractionation labelling may be more representative of biological conditions than analysis with a denaturing gel or on surfaces.

In some embodiments, the fluorescent labelling of the two or more components in the polydisperse sample can happen after the separation of two or more components from each fraction. This can be advantageous as the components are fractionated and can be separated based on its intrinsic biophysical properties.

In some embodiments, the processing step comprises the step of adding an additive into each fraction. Additives added into each fraction containing the component can be advantageous as additive can be used to prevent adhesion. For example, additives can be used to prevent components adhering to each other within the same fraction and/or it can be used to prevent components adhering to a surface. Alternatively, additives can be added to prevent any dissociation of particle complexes after the fractionation process.

In some embodiments, the processing step comprises the step of concentrating the components in each fraction using a pull down assay or immunoprecipitation.

In some embodiments, the processing step comprises adding a magnetic bead in the pull down assay in which the magnetic bead is functionalised to have a specific affinity to each or a subset of the components in each fraction.

The magnetic beads of the pull down assay may be functionalised to have a specific affinity to each or a subset of the components in each fraction. The fractions can be mixed with the magnetic beads and the components of interest in each fraction can bind to the beads. The beads can then be immobilised onto a surface and the unbound components can be washed away. In some embodiments, a washing step may be provided to remove unbound components such as the use of a buffer solution. During this step, the pulled-down components may be labelled by a label. There are different classes of labels such as fluorescent labels, chemoluminescent labels, absorption labels, other electro-magnetic labels, mass labels, radioactive labels, enzyme labels. For each of those classes there are multiple types of labelling agents such as specific or immune-labels (labelled affinity probes or immunoprobes, for example antibody, antibody fragment, aptamer, nanobody, darpin), unspecific labels such as labels attached at an amine or a cysteine group, C-terminus or N-terminus of the peptide, covalently bound labels, hydrophobically interacting labels, environmentally sensitive labels, latent labels, nanoparticle labels that can be configured to enhance scattering or absorption cross sections and isotope labels. A strong advantage of labelling at this point is that components or the fractions that are not of interest have been washed away and are thus not part of the labelling reaction. In particular, this allows for the use of non-specific labels.

The components can then be eluted with a potentially small volume of solution or buffer at certain time points and collected into fractions. The collected fractions would contain a higher concentration of the component if the volume of the eluent is smaller than the original solution volume. This additional processing step results in the pre-concentration of the components in each collected fraction.

Additionally or alternatively, the pull-down or immunoprecipitation assay can also be performed with non-magnetic beads that are pulled down by gravitational, including centrifugal, forces.

Additionally or alternatively, the processing step of each fraction may also comprise one or more of the following steps; a buffer exchange; or the addition salts for pre-concentration of the fraction; or the addition of anti-adhesion agents such as surfactants, polymers, proteins or mixtures; or the addition of molecules suppressing electro-osmotic flow. For example, anti-adhesion agents may include, but is not limited to, one or more of the following; Triton, Tween20 i.e. Polysorbate 20, SDS, Brij, PEG, PVA, PVP, PMOXA, HPC, HPMC BSA, HSA, milk protein, glycoproteins, Non-fat dry milk, fish gelatin and/or serum. In addition, one or more of the following; PVP, PVA, HPC, HPMC may also suppress electro-osmotic flow.

In some embodiments, the two or more components in each fraction prior to the processing step can be measured in their native state.

The separation of the components within the separation channel can be based on, but is not limited to, one or more of the following: electrophoresis; liquid chromatography such as hydrophobic interaction, size-exclusion, ion-exchange, affinity chromatography; hydrodynamic separation; asymmetric flow-field flow fractionation; gas chromatography; diffusion; magnetically using a magnetic field diffusiophoresis or the separation of components can be based on a pH gradient.

Additionally or alternatively, the separation of components in the separation channel can also be based on a non-direct separation technique such as a labelling affinity technique i.e. multicolour labelling of components, and then separate the different value of fraction by viewing at different wavelength.

Additionally or alternatively, the separation of components in each fraction can be based on mass spectrometry techniques.

The high-resolution separation of each fraction can be performed in series or in parallel (at the same time). The result of this separation step can be transient, and a measurement is taken at the outlet. As this is a high-resolution separation of the component, a high number of very small separation fractions should be collected quickly to keep the separated species separated.

In some embodiments, the distribution of the component within the separation channel can be created electrophoretically through the application of an electric field.

An example of an electrophoretic technique that can be applied to distribute the components within the separation channel can be capillary electrophoresis.

In some embodiments, the distribution of the component within the separation channel can be created magnetically through the application of a magnetic field.

In some embodiments, the distribution of the component within the separation channel can be created using one or more of the following techniques; liquid and/or gas chromatography techniques such as size-exclusion chromatography (SEC), ion-exchange chromatography (IEC), reverse-phase chromatography, hydrophobic interaction chromatography, Fast protein liquid chromatography, Mass Spec or LC-MS; asymmetric flow-field flow fractionation (AF4) and/or hydrodynamic separation.

In some embodiments, the distribution of the components within the separation channel can be created by diffusion.

In some embodiments, the two or more components are in a native state prior to and/or during fractionation of the combined fluid flow. It can be advantageous to provide two or more components in their native state because fractionation, separation and analysis can be done based on the inherent biophysical properties of the component.

In some embodiments, the separation of the two or more components from each fraction can occur in solution. This removes the requirement of using techniques that require an immobilisation of a binding partner on a surface, which can often distort separation and analysis. Thus, by separation two or more components from each fraction in solution ensure that the components are separated in their natural form.

In some embodiments, the detection of at least two or more components of each fraction can be done using by optical detection techniques.

In some embodiments, the detection of at least two or more components of each fraction can be done using by fluorescence.

Additionally or alternatively, the detection of at least two or more components of each fraction can be, but is not limited to, scattering detection techniques, absorbance detection techniques, charge or conductivity detection, electrochemical detection, chemiluminescent detection, or mass spectrometry.

In some embodiments, the component is a biomolecule or a biological or chemical molecule or particle. The method of the present invention can be used to characterise a component such as a biomolecule where the biomolecule is a protein, a polypeptide, a peptide, an amino acid, an exosome or a lipid, an antibody or an antibody fragment thereof, a nucleotide or polynucleotide such as DNA or DNA piece, RNA or mRNA, or a polysaccharide. The component may be a complex of different biomolecules.

In some embodiments, the two or more components may be different isoforms of the same biomolecules. The different isoforms may be detected by the same affinity probe or immunoprobe.

In some embodiments, the biomolecule is a multi-biomolecule complex. In some embodiments, the biomolecule may be an affinity reagent such as an antibody, a single domain antibody or an aptamer.

In some embodiments, the multi-biomolecule complex comprises an antibody and an antigen. In some embodiments, one biomolecule in the multi-biomolecule complex can be labelled or at least two or more biomolecules can be labelled. In some embodiments, the antibody can be labelled in order to detect other biomolecules of interest. In some cases, due to the fact that the antibody of interest may be mixed with other antibodies in a sample, it is preferable for the antigen to be labelled.

In some embodiments, the sample fluid can be any type of human fluid or sample. In some embodiments, the sample fluid can be a human serum comprising the biomolecule. In some embodiments, the sample may be a saliva sample or a cell lysate sample. The invention will now be further and more particularly described, by way of example only, and with reference to the accompanying drawings, in which:
Figure 1 provides an illustration of the device for carrying out a method of analysing a biophysical property of a component in a sample according to the present invention;
Figure 2 provides a schematic of the method according to the method of the present invention;
Figure 3A shows a chromatogram of the measured fluorescence intensity of two fractions after separation;
Figure 3B shows a 2D plot of the measured fluorescence intensities according to Figure 3A;
Figure 4 provides a graphical representation of the hydrodynamic radii as extracted from the separated fluorescence curves;
Figure 5 shows a schematic of the process of post-fractionation labelling and plots of different isoforms using the same affinity probe with a reference measurement of the affinity probe on its own; and
Figures 6A and 6B show the separation profiles of the fractions versus effective velocity before and after correction for the affinity probe signal.

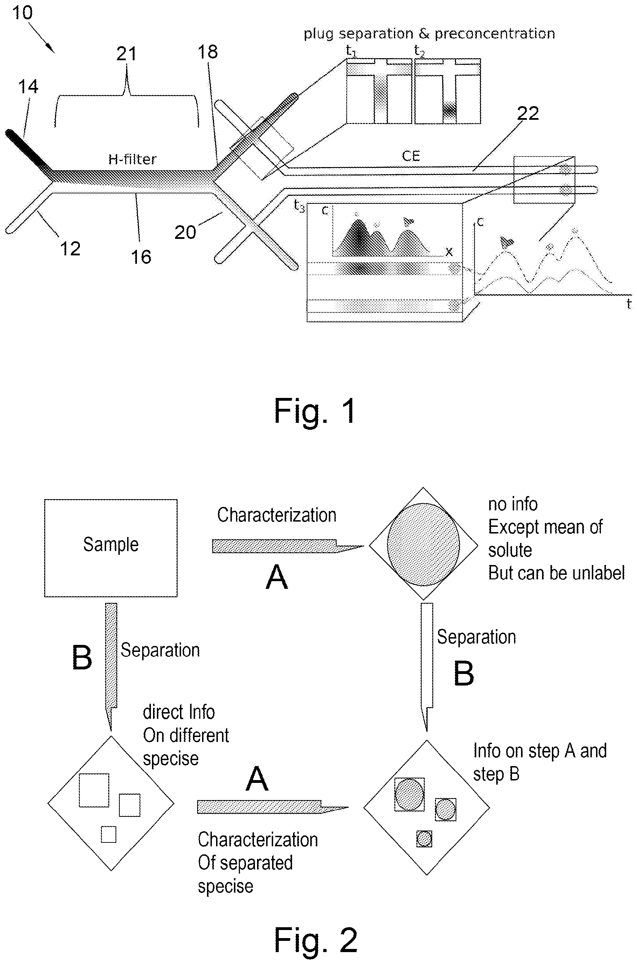

Referring to Figure 1, there is provided a microfluidic device **10** for carrying out a method of determining a biophysical property, for example the diffusion coefficient, of one or more components in a polydisperse sample. The method and device of the present invention as disclosed herein may also be used for determining a biophysical property, for example the diffusion coefficient, of two or more components in a polydisperse sample.

The device **10** comprises an auxiliary channel **12** for an auxiliary fluid, such as buffer, and a sample channel **14** for a fluid comprising the polydisperse sample. The polydisperse sample comprises at least one or more components which may be biomolecules. The method of the present invention further comprises the step of introducing an auxiliary fluid flow into a fractionation channel **16.** The polydisperse sample comprising one or more components can also be introduced into the fractionation channel **16.** The sample and the auxiliary fluids can be combined in the fractionation channel **16** to create a combined flow.

The combined flow is fractionated into two or more fractions **18, 20** by diffusion. Two or more components from each fraction can be separated by creating a distribution of the components within a separation channel **22.**

Use of a microfluidic device **10** provides a means to reduce the movement of components to advection (following the flow direction) and diffusion (isotropic). An H-filter **21** is provided as part of microfluidic device **10,** as indicated in Figure 1, to allow two fluid flows of different solutions to join into the fractionation channel **16.** The H-filter **21** also has two fractions **18, 20** at one end of the fractionation channel **16** where the combined flows can be split into each fraction **18, 20.** The only mixing which occurs between the two solutions in the fractionation channel **16** is due to diffusion perpendicular to the flow direction.

The collection of solution can be done after the fractionation step in two fractions **18, 20.** Each fraction contains a fraction of the initial concentration of particles depending on the diffusion coefficient. The diffusion coefficient is related to the size of the particle (hydrodynamic radius).

This information is hidden in a heterogeneous sample, as a direct measurement will give an average of the hydrodynamic radius of the entire sample. Once the different fractions are collected, the components in each fraction can be labelled with an affinity probe and/or an immunoprobe in the solution. This step is preferably not performed prior to the H-filter as the probe will change the size of the particles within the solution.

In a subsequent step, the components in each of the fractions can be separated in solution using capillary electrophoresis in each of the separation channels **22,** either with one capillary being used to separate each fraction, or two distinct capillaries being used, one for each fraction. The optical detector records each separated, in solution, component sequentially in each fraction, although two or more capillaries can be observed in parallel where appropriate.

Electrophoresis is a technique for separation of nucleic acids, peptides, and cells. Gel electrophoresis, in which analyte charge-to-size ratio is assessed via retardation in a solid matrix upon the application of an electric field, is the most common technique, though this is not well suited for the study of weak protein association events as the act of matrix sieving itself can disrupt interactions. Capillary Electrophoresis (CE) involves the temporal separation of analytes based on their differential electrophoretic mobility and electroosmotic flow throughout a channel. In Free-Flow Electrophoresis (FFE), the sample moves throughout a planar channel through pressure or displacement-driven flow, and separation upon application of an electric field is perpendicular to the direction of flow. Because FFE is a steady-state technique, injection and separation are performed continuously. Microfluidic Free-Flow Electrophoresis (MFFE), a microfluidic miniaturization of FFE, has the advantage of improving separation resolution by reducing the effect of Joule heating and facile online integration with other separation techniques.

The separation of the components using capillary electrophoresis is made using the mobility differences of the two or more components such as different protein complexes with labelled affinity probes and/or immunoprobes. Different peaks should appear in function of the mobility of the different type of biomolecule within the solution.

The separation step may be performed under native conditions to allow an understanding of the component and its environment, including its relationship with other components in a multicomponent mixture. The subsequent analysis may include denaturing and labelling steps to permit accurate identification and characterisation of separated component.

At a point downstream from the separation channels **22,** a characteristic of each the two or more components in each fraction can be detected using high resolution or high sensitivity detection techniques such as a fluorescence detection technique by means of a photodiode, photomultiplier or a high resolution camera.

The characteristic of each component in each fraction can be compared in order to determine the biophysical property of each of the two or more components in the polydisperse sample.

The fractionation of the components in the polydisperse sample can be entropy driven fractionation. An example of entropy driven fractionation can be diffusional sizing. At the fractionation stage, the sample does not need to be completely separated, but a difference in relative or absolute concentration of the components should exist. The fractions are usually poorly separated, and most components are usually present in every fraction, at different concentrations. An entropy-driven separation has the property that the potential energy in each fraction is the same. The presence of a single molecule in that fraction therefore does not give any information about it and only statistical analysis leads to useful information. It is therefore necessary to analyse the difference in relative concentrations. Entropy driven fractionation methods are usually not used for separation, but for measurement. Due to the lack of separation power, the diffusion coefficient can be measured accurately for a monodisperse sample but struggles when polydispersity is involved. Collecting fractions is usually easy; as the experiment is typically time invariant and the collection is done perpendicular to the flow.

Each fraction is collected separately. At this point, the biophysical properties of the components do not need to be extractable. The fractions can then be further processed in a processing step as described below. Some processing can be applied to each fraction, such as labelling, after the fractionation step and prior to the separation step. The labelling of components can be fluorescence labelling such as latent or non-latent labelling or use of affinity probes and/or immunoprobes.

Quantitative labelling procedures, such as the fluorescent labelling procedures described herein, allow the concentration of a component to be directly determined from the recorded analytical signal.

In some cases, non-latent labels may have the same fluorescent intensity if they are attached to the component of interest or not. For example: if approximately 30% of the labels are attached to the component and approximately 70% are unattached to the component, there would be 100% fluorescence. This is the same signal for any attached percentage. Therefore, the signal does not provide any information. With the method of the present invention, the attached and unattached labels would be separated in different separated species. Therefore, the detection of one peak with 30% fluorescence and there shall be a further detection of another peak with 70% fluorescence. Thus, the method of the present invention enables the effective detection of non-latent labels attached to the components and the subsequent analysis of each separated component in each fraction.

Additionally or alternatively, it is also possible to add into each fraction solutes or solvents ions/salts to perform pre-concentration steps i.e. increase the effective concentration of the component in the collected sample.

The added solutes/solvents can affect the molecules or particles or concentrations in each fraction. Therefore, performing this step after fractionation step can help preserve the related biophysical properties of the component, even if the proteins are modified.

Another example would be to use a concentration assay to increase the concentration of the component in each fraction, such as a pull-down assay of immunoprecipitation. The pull-down assay or immunoprecipitation may be performed using magnetic beads for collection. Typically, upon elution from the concentration set up the component would be altered due to denaturation, digestion, or being bound to a capture probe.

Fractions can be separated using a number of high-resolution separation techniques. This is especially important for labelled immunoprobes for example. The separation can be based on, but not limited to, a gradient such as electrophoresis, diffusiophoresis, pH gradient, magnetic field and/or a non-direct separation technique like a labelling affinity technique.

The high-resolution separation of each fraction can be performed in series or in parallel (at the same time). The result of this separation step can be transient, and a measurement is taken at the outlet. Separation fractions can also be collected and then measured later. A high number of very small separation fractions may be collected quickly to keep the separated species separated. The measurement can be done by different technique, Detection of the component in each fraction can be done optically for example using fluorescence (autofluorescence or with help of optional labelling step), absorbance detection techniques, scattering detection techniques, electromagnetically, electrochemically and/or mass measurement.

There may be a detection zone for detecting of the components. The detection can occur downstream from the separation channel. The detection zone may comprise the analytical or detection devices for analysing at least one component in each fraction.

The detection or analytical device is not particularly limited and includes those device that are suitable for use with flow apparatus, and particularly microfluidic devices. A plurality of analytical devices may be provided to determine different physical and chemical characteristics of the component. The analytical devices may be arranged sequentially or in parallel. In some examples, the analytical device can be a fluorimeter or a dry mass measuring device, such as a quartz crystal microbalance.

The separation result from each fraction is compared. This comparison enables the extraction of the biophysical properties, such as the hydrodynamic radius, of each separated component from the fractionation step. In some instances, after appropriate mixing and reaction, the components in each fraction can be compared.

The components can be analysed by calculating the size by cross-correlating diffused and undiffused curves with an optimised scaling factor. The scaling factor can be converted to hydrodynamic radius via finite-element modelling or analytical calculations (depending on the H-filter geometry).

The flow rate of each flow in the sample channel, auxiliary channel, fractionation channel and/or the separation channel can be maintained at a substantially constant level during the fractionation, separation and analysis steps. The fractionation, separation and analysis steps may be undertaken when a stable flow is established in the channels of each section.

The component may be or comprise a polypeptide, a polynucleotide or a polysaccharide. In some embodiments, the component is or comprises a polypeptide. In some embodiments, the component is or comprises a protein. The component may be part of a multicomponent complex. The separation step may therefore be used to at least partially separate the component from other components. For example, the techniques described herein allow for separation based on size or charge-to size ratio, amongst others. In some embodiments, the multicomponent complex comprises agglomerations of components, including proteins, such as monomer, dimer and trimer species, or other higher order agglomerations. Thus, the techniques described herein may be used to separate and analyse protein-protein interactions.

Referring to Figure 2, there is shown a schematic of the method of the present invention. The first step is to fractionate the components in the polydisperse sample using diffusive sizing. The information is therefore not accessible after this first fractionation step, even if this step is the measurement step.

To separate the components in the polydisperse sample into its separated species, a high-resolution separation step splits a small volume into a lot of separation fractions. The volume of each separation fraction is therefore extremely small. Doing any processing after separation is therefore extremely difficult. Due to the small volumes following the high-resolution separation, the measurement and detection preferably happens in line after the separation.

The implementation of the method as described in the present invention can be easier because the systems can be decoupled when commuted.

The fractionation-based measurement technique splits the sample into a two or more fractions, and typically less than or equal to five fractions. The volumes are therefore similar to the initial volume and can therefore be collected and processed. The components in the sample can then be measured in its native state, but generally, the components in the sample would require modification e.g. add a fluorescent label or a charged bead to help with the separation.

This commutation between the fractionation step and the separation step allows a user to modify the solution after the fraction-based measurement technique and thus allows measurement of the native state of each separated component in the polydisperse sample.

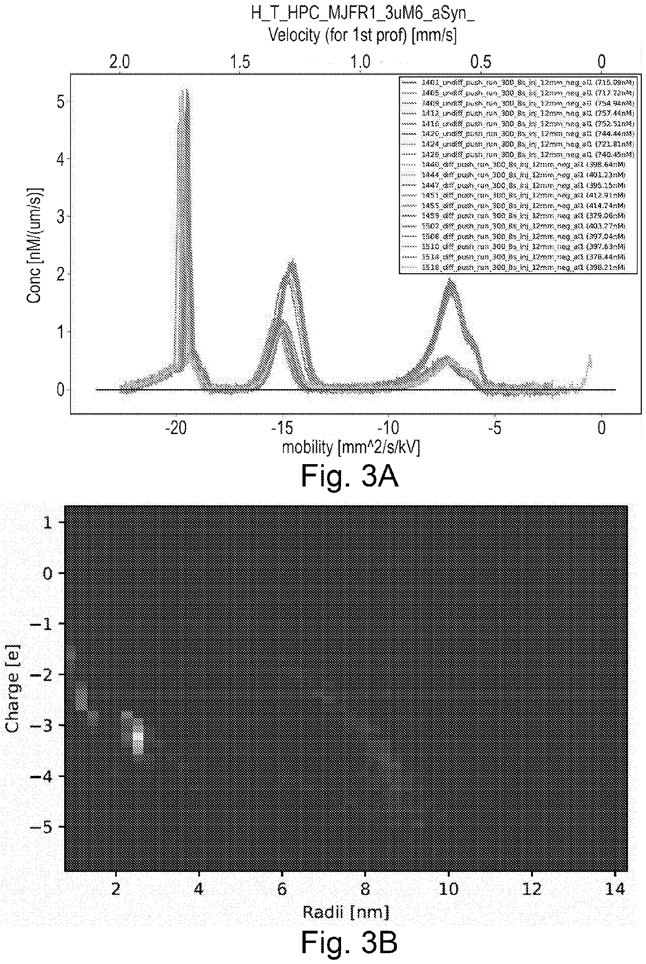

Referring to Figure 3A, the labelled separated components in each fraction can be separated in solution using capillary electrophoresis. The separated components contained in the solution can be recorded when it arrives at an optical detector located downstream from the separation channel. Separation by capillary electrophoresis can be made using the mobility of the system protein and labelled affinity probes and/or immunoprobes. Different peaks should appear in function of the mobility of the different type of protein within the solution, as shown in Figure 3A.

As shown in Figure 3A, there a graph showing the fluorescence intensity of two fractions after performing capillary electrophoresis. The un-diffused fraction is the set of graphs with the higher intensity; the diffused fraction is the set with lower intensity. The size of each component can be determined by comparing the relative intensities of each peak. From left to right the peaks are free Alexa488-fluorophores, BSA, MJFR1-aSyn immunocomplex. Figure 3B shows a 2D plot of charge vs radii of the separated components as described in Figure 3A.

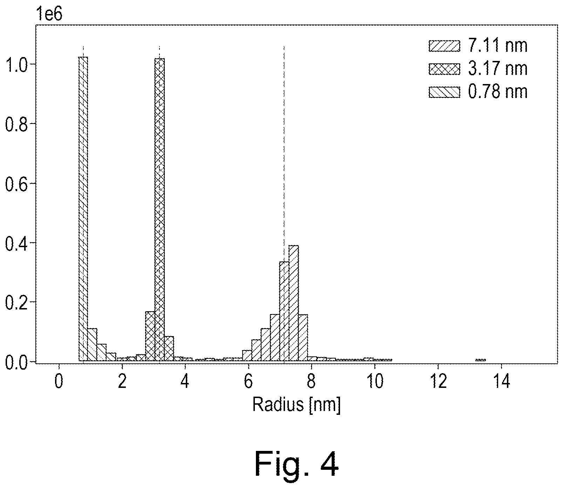

Referring to Figure 4, there is shown a representation of the hydrodynamic radii as extracted from the separated fluorescence curves, segmented into three regions (one per peak). The dashed lines are computed by cross-correlating the diffused and undiffused fractions with a scaling factor, and that scaling factor is converted to a hydrodynamic radius via finite-element modelling. The histograms are computed by aligning each peak and computing the ratio of diffused and undiffused peaks for every measurement point, weighted by the fluorescence intensity at that point.

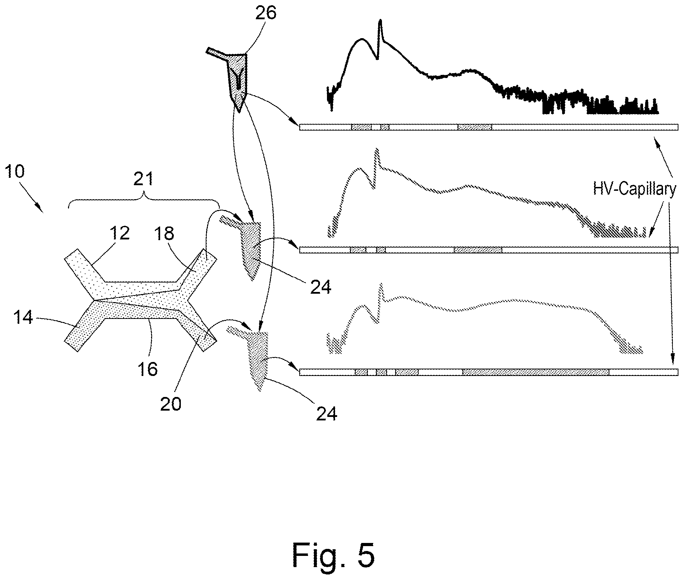

Figure 5 shows a schematic for post-fractionation labelling and a number of plots for the different isoforms of the same biomolecule. The different isoforms can be detected using the same affinity probe or immunoprobe. The plots can be analysed in order to extract the biophysical properties of each isoforms, such as the size of the different isoforms and/or the mobility of the complex. Referring to Figure 5, there is provided a mixture of alpha synuclein monomers and oligomers in a horse serum solution to simulate a human patient sample.

The sample is loaded onto the microfluidic device **10** for carrying out a method of determining a biophysical property, for example the diffusion coefficient, of one or more components in a polydisperse sample.

The device **10** comprises an auxiliary channel **12** for an auxiliary fluid, such as buffer, and a sample channel **14** for a fluid comprising the polydisperse sample. The polydisperse sample comprises at least one or more components which may be biomolecules. The method of the present invention further comprises the step of introducing an auxiliary fluid flow into a fractionation channel **16.** The polydisperse sample comprising one or more components can also be introduced into the fractionation channel **16.** The sample and the auxiliary fluids can be combined in the fractionation channel **16** to create a combined flow. The combined flow can be fractionated into two or more fractions **18, 20** by diffusive sizing.

An H-filter **21** is provided as part of microfluidic device **10,** as indicated in Figure 5, to allow two fluid flows of different solutions to join into the fractionation channel **16.** The H-filter **21** also has two fractions **18, 20** at one end of the fractionation channel **16** where the combined flows can be split into each fraction **18, 20.** The only mixing which occurs between the two solutions in the fractionation channel **16** is due to diffusion perpendicular to the flow direction. The fractions can be collected using any means such as a tube **24,** as indicated in Figure 5.

The fractions can then be incubated with labelled antibodies **26,** as indicated in Figure 5. Subsequently, the separation and analysis of the fractions can be carried out by performing capillary electrophoresis on both fractions and on the antibody. As a result, the biophysical properties of each isoform can be extracted.

In some examples, the fractionation channel **16** i.e. the diffusion chamber can be 10mm x 70um x 40um. The diffusion flow rate can be 33ul/h. The two or more capillary channels for the two or more fractions **18, 20** can be 30um x 5um x 17cm. The reading position can be at 10 mm, at 40mm, at 100mm or at 165mm. Resolution will be optimised by having the detection position close to the total channel length. However, this must be balanced with the time taken for the measurement as a shorter detection length can be used to speed up the measurement. The applied voltage on the two or more fractions or capillary channels can be 15kV. The sample can move along the two or more fractions **18, 20** at approximately 200um/s or approximately 1200um/s or approximately 2500um/s or approximately 10000um/s.

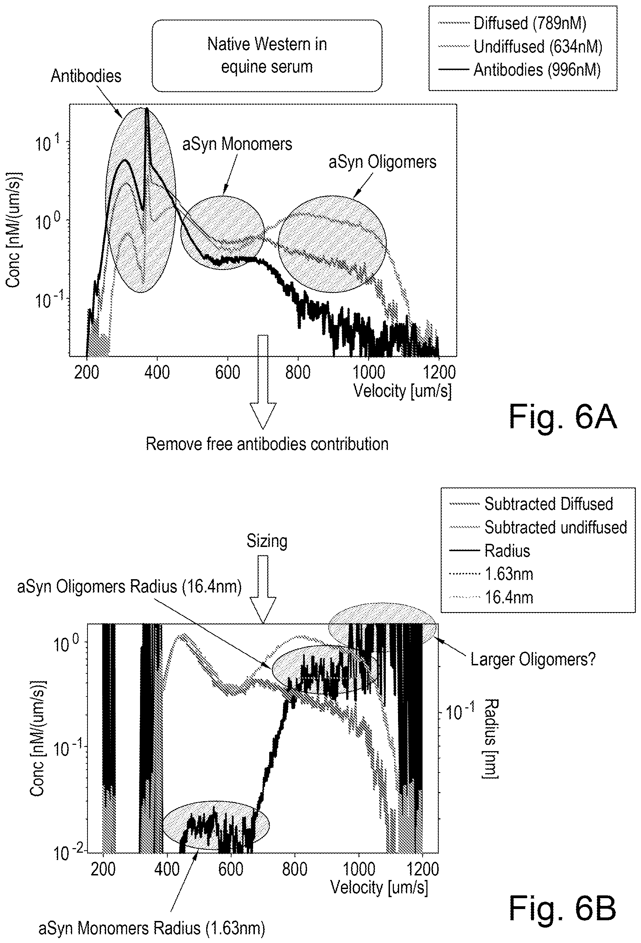

Figure 6A provides a plot that shows the profiles of the fractions versus effective velocity. The antibody shape, as shown in Figure 6A, is removed from both of the fractions' plot profiles, as shown in Figure 6B in order to utilize only the signal from the immune-complexes for analysis of the hydrodynamic radius. The ratio of processed profiles can then be used to obtain the size of the isoform. Additionally or alternatively, the velocity of the isoforms can also yield the mobility of the complexes, such as the mobility of the immuno-complex.

## Claims

1. A method of determining the diffusion coefficient of one or more components in a polydisperse sample, the method comprising the following steps in the following order:
introducing an auxiliary fluid flow into a fractionation channel;
introducing the polydisperse sample comprising one or more components into the fractionation channel;
allowing the sample fluid and the auxiliary fluid to create a combined flow;
fractionating the combined flow into two or more fractions by diffusive sizing;
adding into each fraction solutes or solvents selected from:
affinity probes or immunoprobes for labelling;
ions or salts to perform pre-concentration;
additives to reduce risk of sample adhesion to the wall of the channel of the microfluidic device;
additives to modify the amount of the electro-osmotic flow within an electrophoresis separation channel;
additives to enhance the quantum yield or lifetime of a fluorophore;
separating two or more components from each fraction by creating a distribution of the components within a separation channel;
processing the components in one or more of the fractions to separate and detect components added before or during the method, wherein said added components include:
a blank or auxiliary fluid;
any additive such as immunoprobes or affinity probes;
ions or salts to perform pre-concentration;
additives to modify the amount of the electro-osmotic flow within an electrophoresis separation channel;
additives to enhance the quantum yield or lifetime of a fluorophore;
detecting a characteristic of each of the two or more components in each fraction; and
comparing the characteristic of each component in each fraction in order to determine the diffusion coefficient of each of the one or more components in the polydisperse sample

2. The method according to claim 1, further comprising the step of processing the components in each fraction.

3. The method according to claim 1, further comprising the step of processing the component in the polydisperse sample prior to fractionating the combined flow.

4. The method according to any one of the preceding claims, wherein the processing step comprises the step of labelling the components in each fraction.

5. The method according to claim 4, wherein the labelling of the component comprises a labelled affinity probe.

6. The method according to claim 5, wherein the affinity probe is an antibody, antibody fragment, a nanobody, an aptamer or a darpin.

7. The method according to any one of the preceding claims, wherein the label of the components in each fraction is a fluorescent label.

8. The method according to claim 7, wherein the label of the components in each fraction can be a non-latent fluorescence label.

9. The method according to any one of the preceding claims, wherein the processing step comprises the step of adding an additive into each fraction.

10. The method according to any one of the preceding claims, wherein the processing step comprises the step of concentrating the components in each fraction using a pull down assay or immune-precipitation; and wherein the processing step comprises adding a magnetic bead in the pull down assay in which the magnetic bead is functionalised to have a specific affinity to each or a subset of the components in each fraction.

11. The method according to any one of the preceding claims, wherein the distribution of the component within the separation channel is created electrophoretically through the application of an electric field and wherein the distribution of the component within the separation channel is created by capillary electrophoresis.

12. The method according to any one of the preceding claims, wherein the two or more components are in a native state prior to and/or during fractionation of the combined fluid flow.

13. The method according to any one of the preceding claims, wherein the separation of the two or more components from each fraction occurs in solution.

14. The method according to claim 7, wherein the detection of at least two or more components of each fraction is by fluorescence.

15. The method according to any one of the preceding claims, wherein at least one component is a biomolecule such as an antibody, a polypeptide, a polynucleotide or a polysaccharide an antibody fragment thereof or a multi-biomolecule complex which may optionally comprise an antibody and an antigen.

## Patentansprüche

1. Verfahren zum Bestimmen des Diffusionskoeffizienten von einer oder mehreren Komponenten in einer polydispersen Probe, wobei das Verfahren die folgenden Schritte in der folgenden Reihenfolge umfasst:
Einführen eines Hilfsfluidflusses in einen Fraktionierungskanal;
Einführen der polydispersen Probe, die eine oder mehrere Komponenten umfasst, in den Fraktionierungskanal;
Ermöglichen, dass das Probenfluid und das Hilfsfluid einen kombinierten Fluss erzeugen;
Fraktionieren des kombinierten Flusses in zwei oder mehr Fraktionen durch diffusive Größenbestimmung;
Hinzufügen von gelösten Stoffen oder Lösungsmitteln ausgewählt aus Folgendem zu jeder Fraktion:
Affinitätssonden oder Immunsonden zum Markieren;
Ionen oder Salzen, um Vorkonzentration durchzuführen;
Additiven, um Risiko von Probenanhaftung an der Wand des Kanals der mikrofluidischen Vorrichtung zu reduzieren;
Additiven, um die Menge des elektroosmotischen Flusses innerhalb eines Elektrophoresetrennkanals zu modifizieren;
Additiven, um die Quantenausbeute oder Lebensdauer eines Fluorophors zu verbessern;
Trennen von zwei oder mehr Komponenten aus jeder Fraktion durch Erzeugen einer Verteilung der Komponenten innerhalb eines Trennkanals;
Verarbeiten der Komponenten in einer oder mehreren Fraktionen, um Komponenten, die vor oder während des Verfahrens hinzugefügt werden, zu trennen und zu erfassen, wobei die hinzugefügten Komponenten Folgendes beinhalten:
ein Blind- oder Hilfsfluid;
beliebiges Additiv wie Immunsonden oder Affinitätssonden;
Ionen oder Salze, um Vorkonzentration durchzuführen;
Additive, um die Menge des elektroosmotischen Flusses innerhalb eines Elektrophoresetrennkanals zu modifizieren;
Additive, um die Quantenausbeute oder Lebensdauer eines Fluorophors zu verbessern;
Erfassen einer Eigenschaft von jeder von den zwei oder mehr Komponenten in jeder Fraktion; und
Vergleichen der Eigenschaft jeder Komponente in jeder Fraktion, um den Diffusionskoeffizienten von jeder von der einen oder den mehreren Komponenten in der polydispersen Probe zu bestimmen.

2. Verfahren nach Anspruch 1, ferner umfassend den Schritt des Verarbeitens der Komponenten in jeder Fraktion.

3. Verfahren nach Anspruch 1, ferner umfassend den Schritt des Verarbeitens der Komponente in der polydispersen Probe vor dem Fraktionieren des kombinierten Flusses.

4. Verfahren nach einem der vorhergehenden Ansprüche, wobei der Verarbeitungsschritt den Schritt des Markierens der Komponenten in jeder Fraktion umfasst.

5. Verfahren nach Anspruch 4, wobei das Markieren der Komponente eine markierte Affinitätssonde umfasst.

6. Verfahren nach Anspruch 5, wobei die Affinitätssonde ein Antikörper, Antikörperfragment, ein Nanobody, ein Aptamer oder ein Darpin ist.

7. Verfahren nach einem der vorhergehenden Ansprüche, wobei die Markierung der Komponenten in jeder Fraktion eine fluoreszierende Markierung ist.

8. Verfahren nach Anspruch 7, wobei die Markierung der Komponenten in jeder Fraktion eine nicht-latente Fluoreszenzmarkierung sein kann.

9. Verfahren nach einem der vorhergehenden Ansprüche, wobei der Verarbeitungsschritt den Schritt des Hinzufügens eines Additivs zu jeder Fraktion umfasst.

10. Verfahren nach einem der vorhergehenden Ansprüche, wobei der Verarbeitungsschritt den Schritt des Konzentrierens der Komponenten in jeder Fraktion unter Verwendung eines Pull-Down-Assays oder von Immunpräzipitation umfasst; und wobei der Verarbeitungsschritt Hinzufügen eines magnetischen Kügelchens in dem Pull-Down-Assay umfasst, in dem das magnetische Kügelchen funktionalisiert wird, um eine spezifische Affinität zu jeder oder einer Untergruppe der Komponenten in jeder Fraktion aufzuweisen.

11. Verfahren nach einem der vorhergehenden Ansprüche, wobei die Verteilung der Komponente innerhalb des Trennkanals elektrophoretisch durch die Anwendung eines elektrischen Feldes erzeugt wird und wobei die Verteilung der Komponente innerhalb des Trennkanals durch Kapillarelektrophorese erzeugt wird.

12. Verfahren nach einem der vorhergehenden Ansprüche, wobei die zwei oder mehr Komponenten vor und/oder während Fraktionierung des kombinierten Fluidflusses in einem nativen Zustand sind.

13. Verfahren nach einem der vorhergehenden Ansprüche, wobei die Trennung der zwei oder mehr Komponenten aus jeder Fraktion in Lösung erfolgt.

14. Verfahren nach Anspruch 7, wobei die Erfassung von zumindest zwei oder mehr Komponenten jeder Fraktion durch Fluoreszenz ist.

15. Verfahren nach einem der vorhergehenden Ansprüche, wobei zumindest eine Komponente ein Biomolekül wie ein Antikörper, ein Polypeptid, ein Polynukleotid oder ein Polysaccharid ein Antikörperfragment davon oder ein Multibiomolekülkomplex ist, der optional einen Antikörper und ein Antigen umfassen kann.

## Revendications

1. Procédé de détermination du coefficient de diffusion d'un ou plusieurs composants
dans un échantillon polydispersé, le procédé comprenant les étapes suivantes dans l'ordre suivant :
l'introduction d'un flux de fluide auxiliaire dans un canal de fractionnement ;
l'introduction de l'échantillon polydispersé comprenant un ou plusieurs composants dans le canal de fractionnement ;
l'autorisation au fluide d'échantillon et au fluide auxiliaire de créer un écoulement combiné ;
le fractionnement du flux combiné en deux fractions, ou plus, par dimensionnement de diffusion ;
l'ajout dans chaque fraction de solutés ou solvants choisis parmi :
des capteurs d'affinité ou d'immunocapteurs pour le marquage ;
des ions ou des sels pour réaliser la pré-concentration ;
des additifs pour réduire le risque d'adhésion de l'échantillon à la paroi du canal du dispositif microfluidique ;
des additifs pour modifier la quantité de flux électro-osmotique à l'intérieur d'un canal de séparation par électrophorèse ;
des additifs pour améliorer le rendement quantique ou la durée de vie d'un fluorophore ;
la séparation de deux composants, ou plus, de chaque fraction par la création d'une distribution des composants à l'intérieur d'un canal de séparation ;
le traitement des composants dans une ou plusieurs des fractions pour séparer et détecter des composants ajoutés avant ou durant le procédé, lesdits composants ajoutés comprenant :
un fluide vierge ou auxiliaire ;
tout additif tel que des immunocapteurs ou des capteurs d'affinité ;
des ions ou des sels pour réaliser la pré-concentration ;
des additifs pour modifier la quantité de flux électro-osmotique à l'intérieur d'un canal de séparation par électrophorèse ;
des additifs pour améliorer le rendement quantique ou la durée de vie d'un fluorophore ;
la détection d'une caractéristique de chacun des deux composants, ou plus, dans chaque fraction ; et
la comparaison de la caractéristique de chaque composant dans chaque fraction afin de déterminer le coefficient de diffusion de chacun du ou des composants dans l'échantillon polydispersé.

2. Procédé selon la revendication 1, comprenant en outre l'étape de traitement des composants dans chaque fraction.

3. Procédé selon la revendication 1, comprenant en outre l'étape de traitement du composant dans l'échantillon polydispersé avant de fractionner le flux combiné.

4. Procédé selon l'une quelconque des revendications précédentes, ladite étape de traitement comprenant l'étape de marquage des composants dans chaque fraction.

5. Procédé selon la revendication 4, ledit marquage du composant comprenant un capteur d'affinité marquée.

6. Procédé selon la revendication 5, ledit capteur d'affinité étant un anticorps, un fragment d'anticorps, un nanocorps, un aptamère ou une darpine.

7. Procédé selon l'une quelconque des revendications précédentes, ledit marqueur des composants dans chaque fraction étant un marqueur fluorescent.

8. Procédé selon la revendication 7, ledit marqueur des composants dans chaque fraction pouvant être un marqueur de fluorescence non latente.

9. Procédé selon l'une quelconque des revendications précédentes, ladite étape de traitement comprenant l'étape d'ajout d'un additif dans chaque fraction.

10. Procédé selon l'une quelconque des revendications précédentes, ladite étape de traitement comprenant l'étape de concentration des composants dans chaque fraction en utilisant un essai de type « pull-down » ou à l'aide d'une immunoprécipitation ; et ladite étape de traitement comprenant l'ajout d'une bille magnétique dans l'essai de type « pull-down » dans lequel la bille magnétique est fonctionnalisée pour comporter une affinité spécifique avec chacun ou un sous-ensemble des composants dans chaque fraction.

11. Procédé selon l'une quelconque des revendications précédentes, ladite distribution du composant à l'intérieur du canal de séparation étant créée par électrophorèse à travers l'application d'un champ électrique et ladite distribution du composant à l'intérieur du canal de séparation étant créée par électrophorèse capillaire.

12. Procédé selon l'une quelconque des revendications précédentes, lesdits deux composants, ou plus, étant dans un état natif avant et/ou durant le fractionnement de l'écoulement de fluide combiné.

13. Procédé selon l'une quelconque des revendications précédentes, ladite séparation des deux composants, ou plus, de chaque fraction se produisant en solution.

14. Procédé selon la revendication 7, ladite détection d'au moins deux composants, ou plus, de chaque fraction étant par fluorescence.

15. Procédé selon l'une quelconque des revendications précédentes, au moins un composant étant une biomolécule telle qu'un anticorps, un polypeptide, un polynucléotide ou un polysaccharide, un fragment d'anticorps de celui-ci ou un complexe multibiomolécule qui peut éventuellement comprendre un anticorps et un antigène.
